Europäisches Patentamt

European Patent Office

Office européen des brevets

(19)

(11) Veröffentlichungsnummer: **0 258 586**
**B1**

(12) # EUROPÄISCHE PATENTSCHRIFT

(45) Veröffentlichungstag der Patentschrift:
26.09.90

(51) Int. Cl.⁵: **A61K 7/13**

(21) Anmeldenummer: 87110194.5

(22) Anmeldetag: **15.07.87**

(54) **Oxidationshaarfärbemittel auf der Basis einer gelförmigen Trägermasse und Verfahren zur Färbung von Haaren.**

(30) Priorität: **31.07.86 DE 3625916**

(43) Veröffentlichungstag der Anmeldung:
**09.03.88 Patentblatt 88/10**

(45) Bekanntmachung des Hinweises auf die Patenterteilung:
**26.09.90 Patentblatt 90/39**

(84) Benannte Vertragsstaaten:
**AT BE CH DE ES FR GB GR IT LI NL SE**

(56) Entgegenhaltungen:
**EP-A- 0 188 216**
**FR-A- 2 428 437**
**FR-A- 2 532 174**
**GB-A- 1 287 343**
**GB-A- 2 003 938**
**GB-A- 2 065 177**

(73) Patentinhaber: **Wella Aktiengesellschaft, Berliner Allee 65, D-6100 Darmstadt(DE)**

(72) Erfinder: **Aeby, Johann, Rte. Châtelet 5, CH-1723 Marly(CH)**
Erfinder: **Mager, Herbert, Dr., Beaumont 5, CH-1700 Fribourg(CH)**
Erfinder: **Konrad, Eugen, Mecklenburger Strasse 101, D-6100 Darmstadt(CH)**

**Beschreibung**

Gele spielen als Trägermasse für Haarfarbstoffe eine wichtige Rolle. Sie werden bevorzugt durch den Einsatz von Ölsäure in Kombination mit mit 2 bis 6 Mol Ethylenoxid oxethylierten Nonylphenolen oder Fettalkoholen erhalten Daneben ist üblicherweise in den Gelen Ethanol oder Isopropanol zur Einstellung der Viskosität enthalten. Diese nimmt mit steigendem Alkoholgehalt ab.

Der Einsatz der oben beschriebenen Gele ist weit verbreitet, da sie sehr einfach und sicher mit preisgünstigen Rohstoffen herstellbar sind und weil in der Regel eine gut reproduzierbare Viskosität erhalten wird, welche über einen langen Zeitraum hinweg konstant und lagerbeständig bleibt.

Neben den genannten Vorteilen besitzen die üblichen Gele jedoch auch einige Nachteile. So wird der in den Haarfärbemitteln enthaltene Ammoniak aus den Gelen rasch in die Umgebung abgegeben, wodurch eine Geruchsbelästigung verursacht wird Zudem bewirkt ein Verlust an Ammoniak in dem Haarfärbemittel ein schlechteres Deckvermögen der Farbstoffe, insbesondere auf ergrautem Haar.

Weiterhin färben sich die bekannten Gele nach dem Vermischen mit Wasserstoffperoxid sehr rasch dunkel. Da diese Dunkelfärbung schneller als die Entwicklung des Haarfarbstoffes erfolgt, kann der Verlauf der Haarfärbung nicht direkt beobachtet werden, wodurch der Anwender zu falschen Schlußfolgerungen bezüglich Dauer und Farbtiefe bei der Durchführung der Haarfärbung kommen kann.

Aufgabe der Erfindung war es daher, ein neues Haarfärbe mittel auf der Basis einer gelförmigen Trägermasse mit den oben genannten Vorteilen zur Verfügung zu stellen, worin die Ammoniakabgabe gering ist und bei dem sich die Trägermasse nach der Wasserstoffperoxidzugabe weniger dunkel färbt.

Es wurde nun gefunden, daß durch ein Mittel zur oxidativen Färbung von Haaren auf der Basis einer gelförmigen Trägermasse und eines darin gelösten Farbstoffgemisches, dadurch gekennzeichnet, daß die gelförmige Trägermasse

A) 8 bis 16 Gewichtsprozent Ölsäure,

B) 8 bis 30 Gewichtsprozent mit 2 bis 6 Mol Ethylenoxid oxethyliertes Nonylphenol und/oder mit 2 bis 6 Mol Ethylenoxid oxethylierten Fettalkohol mit 12 bis 20 Kohlenstoffatomen,

C) 6 bis 14 Gewichtsprozent Fettalkohol mit 10 bis 20 Kohlenstoffatomen,

D) 4 bis 18 Gewichtsprozent Ethanol und/oder Isopropanol und

E) 16 bis 70 Gewichtsprozent Wasser,

bezogen auf die Gesamtmenge des Haarfärbemittels, enthält, die gestellte Aufgabe in hervorragender Weise gelöst wird.

In dem neuen Haarfärbemittel sind die Ölsäure vorzugsweise in einer Menge von etwa 10 bis 12 Gewichtsprozent, das mit 2 bis 6 Mol oxethylierte Nonylphenol und der mit 2 bis 6 Mol oxethylierte Fettalkohol vorzugsweise in einer Menge von etwa 12 bis 25 Gewichtsprozent, der Fettalkohol vorzugsweise in einer Menge von etwa 8 bis 12 Gewichtsprozent, das Ethanol und das Isopropanol vorzugsweise in einer Menge von etwa 6 bis 12 Gewichtsprozent sowie das Wasser vorzugsweise in einer Menge von 25 - 50 Gewichtsprozent enthalten.

Daß die gelförmige Trägermasse trotz des hohen Fettalkoholgehaltes transparent bleibt, ist überaschend und war nicht vorauszusehen.

Als geeignetes oxethyliertes Nonylphenol kommt beispielsweise mit 4 Mol Ethylenoxid oxethyliertes Nonylphenol in Betracht.

Der oxethylierte Fettalkohol der Komponente B) besitzt vorzugsweise 16 bis 18 Kohlenstoffatome. Ein geeigneter oxethylierter Fettalkohol ist zum Beispiel mit 2 Mol Ethylenoxid oxethylierter Laurylalkohol.

Beispiele für geeignete Fettalkohole sind Cetylstearylalkohol, welcher auch in Form eines kolloiddispersen Gemisches aus 90 Teilen Cetylstearylalkohol und 10 Teilen Natriumlaurylsulfat, welches von der Firma Henkel unter den Namen Lanette® O und Lanette® W vertrieben wird, eingesetzt werden kann.

Darüber hinaus kann die Trägermasse noch weitere für solche Mittel übliche Zusätze, zum Beispiel mehrwertige Alkohole wie Ethylenglykol, 1,2-Propylenglykol und Glycerin, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Fettsäuretauride, Alkyltrimethylammoniumsalze, Alkylbetaine, Fettsäurealkanolamide, oxethylierte Fettsäureester sowie außerdem Pflegestoffe wie Lanolinderivate, Cholesterin, Pantothensäure und Betain enthalten.

Die erwähnten Bestandteile werden in den für solche Zwecke üblichen Mengen verwendet, zum Beispiel können die Netzmittel und Emulgatoren in Konzentrationen von etwa 0,5 bis 10 Gewichtsprozent und die Pflegestoffe in einer Konzentration von etwa 0,1 bis 5 Gewichtsprozent in den Zubereitungen enthalten sein.

Weiterhin kann die Trägermasse Antioxidantien wie Ascorbinsäure, Resorcin oder Natriumsulfit sowie bis zu 0,1 Gewichtsprozent Parfümöle und bis zu 5,0 Gewichtsprozent Komplexbildner für Schwermetalle, jeweils bezogen auf die Gesamtzusammensetzung des Mittels, enthalten.

Je nach Zusammensetzung kann das erfindungsgemäße Haarfärbemittel schwach sauer, neutral oder alkalisch reagieren. Insbesondere weist es einen pH-Wert im alkalischen Bereich zwischen 8,0 und 11,5 auf, wobei die Einstellung vorzugsweise mit etwa 0,1 bis 5,0 Gewichtsprozent Ammoniak erfolgt. Es können aber auch organische Amine, zum Beispiel Monoethanolamin und Triethanolamin, oder auch anorganische Basen, wie beispielsweise Natrium-hydroxid und Kaliumhydroxid, Verwendung finden.

Das in dem neuen gelförmigen Haarfärbemittel enthaltene Farbstoffgemisch besteht aus mindestens einer Entwicklersubstanz und mindestens einer Kupplersubstanz. Gegebenenfalls können zusätzlich mit sich selbst kuppelnde Farbvorstufen und direkt auf das Haar aufziehende Farbstoffe enthalten sein.

Die Entwickler- und Kupplersubstanzen werden in dem Haarfärbemittel entweder als solche oder in Form ihrer physiologisch unbedenklichen Salze mit anorganischen oder organischen Säuren, zum Beispiel als Chlorid, Sulfat, Phosphat und Acetat eingesetzt.

Von den bekannten Entwicklersubstanzen kommen als Be standteil des neuen Haarfärbemittels vorzugsweise 1,4-Diamino-benzol, 2,5-Diamino-toluol, 2,5-Diamino-benzyl-alkohol, 2-(2'-Hydroxyethyl)-1,4-diamino-benzol und 4-Amino-phenol in Betracht.

Als geeignete Kupplersubstanzen seien beispielsweise Resorcin, 4-Chlor-resorcin, 2,4-Dichlor-resorcin, 2-Methyl-resorcin, 3-Amino-phenol, 5-Amino-2-methyl-phenol, 4-Amino-1,2-methylendioxy-benzol, 4-(2'-Hydroxyethyl)amino-1,2-methylendioxy-benzol, 1-Naphthol, 4-Hydroxy-indol, 2,4-Dihydroxyanisol, 3-Amino-anilin, 2-Amino-4-(2'-Hydroxyethyl)amino-anisol und 2',4'-Dihydroxy-2-phenoxyethanol erwähnt.

Die Gesamtmenge der in dem hier beschriebenen Haarfärbemittel enthaltenen Entwicklersubstanz-Kupplersubstanz-Kombination soll etwa 0,1 bis 6,0 Gewichtsprozent, vorzugsweise etwa 0,5 bis 5,0 Gewichtsprozent betragen.

Zur Erzielung gewisser Farbnuancen können ferner auch übliche direktziehende Farbstoffe, beispielsweise aromatische Nitrofarbstoffe wie 2-Amino-4-nitro-phenol, 2-Amino-6-chlor-4-nitro-phenol, 2-(2'-Hydroxyethyl)amino-4,6-dinitro-phenol, 3-(2',3'-Dihydroxypropyl)amino-4-nitro-trifluormethylbenzol, sowie Disperse Violet 1 (C.I. 61 100) und 1,4,5,8-Tetraamino-anthrachinon enthalten sein.

Die Gesamtmenge des Farbstoffgemisches beträgt etwa 0,1 bis 6,0 Gewichtsprozent, vorzugsweise etwa 0,5 bis 5,0 Gewichtsprozent.

Das erfindungsgemäße Oxidationshaarfärbemittel stellt ein Gemisch aus der gelförmigen Trägermasse und dem Farbstoffgemisch dar.

Bei der Anwendung vermischt man das Haarfärbemittel unmittelbar vor dem Gebrauch etwa im Gewichtsverhältnis 5:1 bis 1:4 mit einem Oxidationsmittel und trägt eine für die Färbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommt hauptsächlich Hydrogenperoxid, beispielsweise als 6-prozentige wäßrige Lösung, beziehungsweise dessen Additionsverbindungen an Harnstoff, Melamin oder Natriumborat in Betracht. Man läßt das Gemisch bei 15 bis 50 Grad Celsius etwa 10 bis 45 Minuten lang, vorzugsweise 30 Minuten lang, auf das Haar einwirken, spült sodann das Färbegemisch mit Wasser aus und trocknet. Gegebenenfalls wird im Anschluß an diese Spülung mit einer schwachen physiologisch verträglichen organischen Säure, wie zum Beispiel Zitronensäure oder Weinsäure, nachgespült.

Das in Verbindung mit dem neuen Oxidationshaarfärbemittel verwendete Hydrogenperoxid kann sowohl in Form einer wäßrigen Lösung als auch in Form einer niedrig- bis mittelviskosen Emulsion vorliegen. In der Regel kommt das emulsionsförmige Hydrogenperoxid zum Einsatz, wenn das Haarfärbemittel mit einer Auftragebürste auf das Haar aufgetragen wird. Falls die Haarfärbung mit einer Auftrageflasche vorgenommen wird, ist die Verwendung von Hydrogenperoxid in wäßriger Lösung bevorzugt. Hierbei wird zunächst das Hydrogenperoxid in der Auftrageflasche vorgelegt. Sodann wird das Haarfärbemittel aus der Tube in die Hydrogenperoxidlösung gedrückt und anschließend durch Schütteln der Auftrageflasche zur anwendungsfertigen Oxidationsfärbemasse gemischt. Der Mischvorgang verläuft rasch und problemlos. Die fertige Oxidationsfärbemasse kann nun durch leichten Druck auf die Auftrageflasche entnommen werden.

Zur Verdickung des in Form einer Emulsion vorliegenden Hydrogenperoxids können beispielsweise etwa 1 bis 2 Gewichtsprozent Cetylstearylalkohol in Kombination mit anionischen Emulgatoren, wie zum Beispiel Lauryl- alkohol-diglykolethersulfat, verwendet werden.

Die erfindungsgemäßen Haarfärbemittel haben die vorteilhafte Eigenschaft, daß sie auch nach längerer Lagerzeit nicht andicken, wodurch sich die Mischbarkeit mit dem Hydrogenperoxid verschlechtern würde.

Die durch die erfindungsgemäße gelförmige Trägermasse gekennzeichneten neuen Haarfärbemittel weisen weitgehend unabhängig von der Art und der Menge der enthaltenen Farbstoffe die gleiche Viskosität auf. Deshalb ist es möglich, für alle Töne einer Haarfärbeserie die gleiche Trägermasse zu verwenden, wodurch sowohl die Handhabung des Haarfärbemittels für den Anwender als auch die Herstellung vereinfacht und somit verbilligt wird.

## BEISPIELE FÜR HAARFÄRBEMITTEL

**Beispiel 1:**    gelförmiges Oxidationshaarfärbemittel

| 12,0 g | Ölsäure, destilliert |
|---|---|
| 25,0 g | mit 4 Mol Ethylenoxid oxethyliertes Nonylphenol |
| 10,0 g | Cetylstearylalkohol |
| 8,0 g | Isopropanol |
| 1,0 g | 1,4-Diamino-benzol |
| 0,8 g | Resorcin |
| 0,1 g | 3-Amino-phenol |
| 0,1 g | 4-Amino-phenol |
| 0,3 g | Ascorbinsäure |
| 10,0 g | Ammoniak, 25-prozentige wäßrige Lösung |
| 32,7 g | Wasser |

100,0 g

Das vorstehend genannte Haarfärbemittel wird im Gewichtsverhältnis 1:2 mit einer wäßrigen, 6-prozentigen Hydrogenperoxidlösung in einer Auftrageflasche durch Schütteln vermischt und die entstandene Haarfärbelösung auf blondiertes oder ergrautes Haar aufgetragen. Nach einer Einwirkungszeit von 20 Minuten bei 40 Grad Celsius ist das Haar mittelblond gefärbt.

**Beispiel 2:** gelförmiges Oxidationshaarfärbemittel

**Beispiel 2:** gelförmiges Oxidationshaarfärbemittel

10,0 g Ölsäure, destilliert

12,0 g mit 2 Mol Ethylenoxid oxethylierter Laurylalkohol

10,0 g Cetylstearylalkohol

8,0 g Isopropanol

0,1 g Natriumsulfit

0,3 g Ascorbinsäure

3,0 g Laurylalkohol-diglycolethersulfat-Natriumsalz, 28-prozentige wäßrige Lösung

2,0 g 1,4-Diamino-benzol

0,4 g 3-Amino-phenol

1,6 g Resorcin

9,0 g Ammoniak, 25-prozentige wäßrige Lösung

43,6 g Wasser

---

100,0 g

Das vorstehend genannte Haarfärbemittel wird im Gewichtsverhältnis 1:1 mit einer Emulsion bestehend aus

1,0 g Cetylstearylalkohol

0,3 g Laurylalkohol-diglycolethersulfat-Natriumsalz, 28-prozentige wäßrige Lösung

98,7 g Hydrogenperoxid, 6-prozentige wäßrige Lösung

---

100,0 g

vermischt und die entstandene viskose Haarfärbemasse mit einem Pinsel auf ergrautes Haar aufgetragen. Nach einer Einwirkungszeit von 20 Minuten bei 40 Grad Celsius ist das Haar dunkelbraun gefärbt.

Die in dieser Anmeldung verwendeten Prozentangaben stellen, soweit nicht anders vermerkt, Gewichtsprozente dar.

**Patentansprüche**

1. Mittel zur oxidativen Färbung von Haaren auf der Basis einer gelförmigen Trägermasse und eines darin gelösten Farbstoffgemisches, dadurch gekennzeichnet, daß die gelförmige Trägermasse
A) 8 bis 16 Gewichtsprozent Ölsäure,
B) 8 bis 30 Gewichtsprozent mit 2 bis 6 Mol Ethylenoxid oxethyliertes Nonylphenol und/oder mit 2 bis 6 Mol Ethylenoxid oxethylierten Fettalkohol mit 12 bis 20 Kohlenstoffatomen,
C) 6 bis 14 Gewichtsprozent Fettalkohol mit 10 bis 20 Kohlenstoffatomen,
D) 4 bis 18 Gewichtsprozent Ethanol und/oder Isopropanol und
E) 16 bis 70 Gewichtsprozent Wasser, bezogen auf die Gesamtmenge des Haarfärbemittels, enthält.
2. Mittel nach Anspruch 1, dadurch gekennzeichnet, daß die Komponente A) in einer Menge von 10 bis 12 Gewichtsprozent enthalten ist.

5

3. Mittel nach den Ansprüchen 1 und 2, dadurch gekennzeichnet, daß die Komponente B) in einer Menge von 12 bis 25 Gewichtsprozent enthalten ist.

4. Mittel nach den Ansprüchen 1 bis 3, dadurch gekennzeichnet, daß die Komponente C) in einer Menge von 8 bis 12 Gewichtsprozent enthalten ist.

5. Mittel nach den Ansprüchen 1 bis 4, dadurch gekennzeichnet, daß die Komponente D) in einer Menge von 6 bis 12 Gewichtsprozent enthalten ist.

6. Mittel nach den Ansprüchen 1 bis 5, dadurch gekennzeichnet, daß die Komponente B) aus mit 4 Mol Ethylenoxid oxethyliertem Nonylphenol und/oder mit 4 Mol Ethylenoxid oxethyliertem Fettalkohol mit 12 bis 20 Kohlenstoffatomen besteht.

7. Mittel nach Anspruch 6, dadurch gekennzeichnet, daß der oxethylierte Fettalkohol 16-18 Kohlenstoffatome besitzt.

8. Mittel nach den Ansprüchen 1 bis 7, dadurch gekennzeichnet, daß das in der Trägermasse gelöste Farbstoffgemisch mindestens eine der Entwicklersubstanzen 4-Amino-phenol, 1,4-Diamino-benzol, 2,5-Diamino-toluol, 2,5-Diamino-benzylalkohol, 3-Methyl-4-amino-phenol und 2-(2′-Hydroxyethyl)-1,4-diamino-benzol enthält.

9. Mittel nach den Ansprüchen 1 bis 8, dadurch gekennzeichnet, daß das in der Trägermasse gelöste Farbstoffgemisch mindestens eine der Kupplersubstanzen Resorcin, 4-Chlor-resorcin, 2-Methyl-resorcin, 3-Amino-phenol, 5-Amino-2-methyl-phenol, 4-Amino-1,2-methylendioxy-benzol, 4-(2′-Hydroxyethyl)amino-1,2-methylendioxy-benzol, 1-Naphthol, 3-Amino-anilin und 2-Amino-4-(2′-hydroxyethyl)amino-anisol enthält.

10. Mittel nach den Ansprüchen 8 und 9, dadurch gekennzeichnet, daß die Gesamtmenge der im Farbstoffgemisch enthaltenen Entwicklersubstanz-Kupplersubstanz-Kombination 0,1 bis 6,0 Gewichtsprozent beträgt.

11. Verfahren zum oxidativen Färben von Haaren, dadurch gekennzeichnet, daß man ein Haarfärbemittel nach den Ansprüchen 1 bis 10 unmittelbar vor dem Gebrauch mit einem Oxidationsmittel, insbesondere Wasserstoffperoxid oder dessen Additionsverbindungen an Harnstoff, Melanin oder Natriumborat, vermischt, anschließend eine für die Haarbehandlung ausreichende Menge dieses Gemisches auf das Haar aufträgt und bei 15 bis 50 Grad Celsius 10 bis 45 Minuten lang auf das Haar einwirken läßt, das Haar mit Wasser spült und sodann trocknet.

**Claims**

1. Agent for the oxidative dyeing of hair, based on a vehicle in the form of a gel and a dye mixture dissolved therein, characterised in that the vehicle in the form of a gel contains
A) 8 to 16% by weight of oleic acid,
B) 8 to 30% by weight of nonylphenol ethoxylated with 2 to 6 mol of ethylene oxide and/or a fatty alcohol with 12 to 20 carbon atoms ethoxylated with 6 mol of ethylene oxide,
C) 6 to 14% by weight of a fatty alcohol with 10 to 20 carbon atoms,
D) 4 to 18% by weight of ethanol and/or isopropanol and
E) 16 to 70% by weight of water, based on the total quantity of hair dye.

2. Agent according to Claim 1, characterised in that component A) is contained therein in a quantity of from 10 to 12% by weight.

3. Agent according to Claims 1 and 2, characterised in that component B) is contained therein in a quantity of from 12 to 25% by weight.

4. Agent according to Claims 1 to 3, characterised in that component C) is contained therein in a quantity of from 8 to 12% by weight.

5. Agent according to Claims 1 to 4, characterised in that component D) is contained therein in a quantity of from 6 to 12% by weight.

6. Agent according to Claims 1 to 5, characterised in that component B) consists of nonylphenol ethoxylated with 4 mol of ethylene oxide and/or a fatty alcohol with 12 to 20 carbon atoms ethoxylated with 4 mol of ethylene oxide.

7. Agent according to Claim 6, characterised in that the ethoxylated fatty alcohol has 16 to 18 carbon atoms.

8. Agent according to Claims 1 to 7, characterised in that the dye mixture dissolved in the vehicle contains at least one of the developer substances, 4-amino-phenol, 1,4-diamino-benzene, 2,5-diamino-toluene, 2,5-diaminobenzyl alcohol, 3-methyl-4-amino-phenol and 2-(2′-hydroxyethyl)-1,4-diamino-benzene.

9. Agent according to Claims 1 to 8, characterised in that the dye mixture dissolved in the vehicle contains at least one of the coupler substances, resorcinol, 4-chloro-resorcinol, 2-methyl-resorcinol, 3-amino-phenol, 5-amino-2-methyl-phenol, 4-amino-1,2-methylenedioxy-benzene, 4-(2′-hydroxyethyl)amino-1,2-methylenedioxy-benzene, 1-napthol, 3-amino-aniline and 2-amino-4-(2′-hydroxyethyl)amino-anisole.

10. Agent according to Claims 8 and 9, characterised in that the total quantity of the combination of developer substance and coupler substance contained in the dye mixture amounts to from 0.1 to 6.0% by weight.

11. Process for the oxidative dyeing of hair, characterised in that a hair dye according to Claims 1 to 10 is mixed immediately before use with an oxidizing agent, in particular hydrogen peroxide or addition compounds thereof with urea, melanine or sodium borate, and a quantity of this mixture sufficient for the treatment of the hair is applied to the hair and left to act on the hair for 10 to 45 minutes at 15 to 50 degrees centigrade and the hair is then rinsed with water and dried.

**Revendications**

1. Produit de coloration des cheveux par oxydation à base d'une matière de support à l'état de gel et d'un mélange de colorants qui y est dissout, caractérisé en ce que la matière de support a l'état de gel renferme:

A) 8 à 16% en poids d'acide oléique;

B) 8 à 30 % en poids de nonylphénol éthoxylé avec 2 à 6 moles d'oxyde d'éthylène et/ou d'alcool gras à 12 à 20 atomes de carbone éthoxylé avec 3 à 6 moles d'oxyde d'éthylène;

C) 6 à 14% en poids d'alcool gras comportant 10 à 20 atomes de carbone;

D) 4 à 18% en poids d'éthanol et/ou d'isopropanol, et

E) 16 à 70% en poids d'eau, les pourcentages se rapportant à la quantité totale du colorant pour les cheveux.

2. Produit selon la revendication 1, caractérisé en ce que le constituant A) est contenu dans une proportion de 10 à 12% en poids.

3. Produit selon les revendications 1 et 2, caractérisé en ce que le constituant B) est contenu dans une proportion de 12 à 25% en poids.

4. Produit selon les revendications 1 à 3, caractérisé en ce que le constituant C) est contenu dans une proportion de 8 à 12% en poids.

5. Produit selon les revendications 1 à 4, caractérisé en ce que le constituant D) est contenu dans une proportion de 6 à 12% en poids.

6. Produit selon les revendications 1 à 5, caractérisé en ce que le constituant B) est constitué par du nonylphénol éthoxylé avec 4 moles d'oxyde d'éthylène et/ou un alcool gras à 12 à 20 atomes de carbone ethoxylé avec 4 moles d'oxyde d'éthylène.

7. Produit selon la revendication 6, caractérisé en ce que l'alcool gras éthoxylé comporte 16 à 18 atomes de carbone.

8. Produit selon les revendications 1 à 7, caractérisé en ce que le mélange de colorants dissout dans la matière de support comprend au moins l'une des substances de développement suivantes: le 4-amino-phénol, le 1,4-diamino-benzène, le 2,5-diamino-toluène, l'alcool 3,5-diamino-benzylique, le 3-méthyl-4-amino-phénol et le 2-(2'-hydroxyéthyl)-1,4-diamino-benzène.

9. Produit selon les revendications 1 à 8, caractérisé en ce que le mélange de colorants dissout dans la matière de support renferme l'une au moins des substances de copulation suivantes: la résorcine, la 4-chloro-résorcine, la 3-méthyl-résorcine, le 3-amino-phénol, le 5-amino-2-méthyl-phénol, le 4-amino-1,2-méthylènedioxy-benzène, le 4-(3'-hydroxyéthyl)-amino-1,2-méthylènedioxy-benzène, le 1-napthol, la 3-amino-aniline et le 2-amino-4-(2'-hydroxyéthyl)-amino-anisole.

10. Produit selon les revendications 8 et 9, caractérisé en ce que la proportion totale de la combinaison développeur-copulateur contenue dans le mélange de colorants est de 0,1 à 6,0 % en poids.

11. Procédé de coloration par oxydation des cheveux, caractérisé en ce qu'on mélange un colorant pour les cheveux selon les revendications 1 à 10, juste avant l'utilisation avec un oxydant, en particulier de l'eau oxygénée ou ses composés d'addition à l'urée, la mélanine ou le borate de sodium, puis l'on applique aux cheveux une quantité de ce mélange suffisante pour le traitement des cheveux et on la laisse agir sur les cheveux 10 à 45 minutes à 15 à 50°C, on rince les cheveux à l'eau puis on les sèche.